# EUROPEAN PATENT APPLICATION

(11) **EP 2 383 014 A1**
(43) Date of publication of application: **02.11.2011**
(21) Application number: 10161437.8
(22) Date of filing: 29.04.2010
(51) Int. Cl.: A61N 1/34

(54) **Transcutaneous electro-stimulation device with a matrix of electrodes**

(71) Applicant: Koninklijke Philips Electronics N.V., 5621 BA Eindhoven (NL)
(72) Inventor: Kolen, Alexander, F., 5600 AE, Eindhoven (NL); Puszka, Agathe, 5600 AE, Eindhoven (NL); Wagemakers, Fremke, 5600 AE, Eindhoven (NL)
(74) Representative: Damen, Daniel Martijn

(57) **Abstract**

A transcutaneous electro-stimulation device comprising a matrix of stimulation electrodes (11) and a electronic circuit. The stimulation electrodes (11) are distributed on an electrode pad (10) and are configured to be applied to a skin area of a human or animal body. The skin area covered by the matrix comprises multiple stimulation responsive surface areas. The electronic circuit is coupled to the stimulation electrodes (11) and is configured to apply a stimulation signal to at least one of the stimulation electrodes (11) in order to stimulate one of the stimulation responsive surface areas. The arrangement of the stimulation electrodes (11) in the matrix is such that a distance between adjacent stimulation electrodes (11) in the matrix is smaller than a diameter of the stimulation responsive surface areas.

## Description

### FIELD OF THE INVENTION

This invention relates to a transcutaneous electro-stimulation device comprising a matrix of stimulation electrodes distributed on an electrode pad being configured to be applied to a skin area of a human or animal body, the skin area covered by the matrix comprising multiple stimulation responsive surface areas, and an electronic circuit being coupled to the stimulation electrodes and being configured to apply a stimulation signal to at least one of the stimulation electrodes in order to stimulate one of the stimulation responsive areas.

### BACKGROUND OF THE INVENTION

Such an electrical stimulation device is for instance used in the fields of functional electro-stimulation (also often indicated as FES) and in the field of pain relief via electrical stimulation signals (also often indicated as transcutaneous electrical nerve stimulation, or in short TENS). In both FES and TENS electrical signals are locally applied to the body of a human or animal so as to excite particular parts of the nervous system. Many clinical reports exist concerning the use of TENS for different types of conditions such as low-back pain, myofascial and arthritic pain, sympathetically mediated pain, bladder incontinence, neurogenic pain, visceral pain and postsurgical pain. TENS is the application of electrical stimulation at the surface of the skin (=transcutaneous), primarily for pain relief. TENS is applied via external surface electrodes with some sort of electrical waveform characterized by frequency, pulse duration and amplitude. Electrical stimulation is also used for other purposes, such as electrical muscle stimulation which causes stimulated muscle tissue to contract.

In particular a small electrical current is locally applied to the skin of the human or animal undergoing electro-stimulation. It has been found that such electro-stimulation has various beneficiary effects. As indicated above, pain relief is one of the known beneficiary effects. Furthermore it has been found that electro-stimulation can activate muscular tissue and may activate the healing of wounds. In order to achieve optimum beneficiary effects, the electrical signals are to be applied to particular, optimum positions on the human or animal skin.

This invention is described for the application of TENS to an arthritic knee, but is not exclusive to this application or body part. When TENS is prescribed to treat the painful knee often the therapist will apply the stimulation electrodes according to a fixed protocol, based on a standard treatment rather than an individually dedicated approach. Stimulation electrodes are positioned around the knee according to a protocol which is not based on the patients' individual needs. Additionally, the patient has to position the stimulation electrodes at home by himself, remembering what the physiotherapist showed/told him. If his pain areas in the joint change, the patient has to find out himself how to target the new pain area or has to go back to the physiotherapist.

### OBJECT OF THE INVENTION

Considering the above, there is a need for an electro-stimulation device according to the opening paragraph, which device allows for stimulation of those areas which may benefit from such stimulation.

### SUMMARY OF THE INVENTION

According to a first aspect of the invention, this object is achieved by a matrix of stimulation electrodes distributed on an electrode pad and being configured to be applied to a skin area of a human or animal body, the skin area covered by the matrix comprising multiple stimulation responsive surface areas, and an electronic circuit being coupled to the stimulation electrodes and being configured to apply a stimulation signal to at least one of the stimulation electrodes in order to stimulate one of the stimulation responsive surface areas, the arrangement of the stimulation electrodes in the matrix being such that a distance between adjacent stimulation electrodes in the matrix is smaller than a diameter of the stimulation responsive surface areas.

It is known that stimulation of specific skin areas may result in an electrophysiological effect at a corresponding part of the body somewhere else. Different stimulation responsive surface areas (in short: stimulation areas) correspond to different 'effect zones' (i.e. the corresponding body part, where the stimulation of the specific skin area has an effect). In known TENS devices, this knowledge is used by doctors applying relatively large electrodes to the correct position on the patient's skin. The correct position of the electrodes depends on the effect zone requiring, e.g., pain relief. As described above, this approach may cause problems, e.g., when later at home the patient has to place the electrodes himself or when the area requiring pain relief changes. According to the invention this problem is solved by using a matrix of electrodes covering multiple stimulation points and an electronic circuit being coupled to the electrodes for applying the stimulation signals to one or more selected electrodes. Correct placement of the electrode pad is guaranteed because the arrangement of the electrodes in the matrix is such that each stimulation area is covered by a substantive part of at least one electrode surface. In order to obtain such coverage, the distance between adjacent electrodes must be smaller than the diameter of the stimulation areas. When the stimulation is covered by parts of two, three or more stimulation electrodes, the coverage per overlapping electrode may be smaller than when only one stimulation electrodes covers the stimulation area.

In a preferred embodiment, the size and arrangement of the electrodes in the matrix is such that irrespective of the precise placement of the electrode pad, the stimulation area is covered by at least a total amount of electrode surface corresponding to the surface area of one of the stimulation electrodes. For example, the stimulation area may be covered by at least one full electrode or by at least two separate halve electrodes.

In a possible pain relief application of the invention, the stimulation electrodes in the matrix are divided into at least two clusters of stimulation electrodes and each cluster is related to a specific pain area. As a result the electronics may be programmed to stimulate a certain cluster in order to cause pain relief in the corresponding pain region. Stimulating a certain cluster means activating at least one stimulation electrode in said cluster. Selection of the electrode(s) to be activated may be done manually or by an algorithm performed by the electronic circuit. Due to physiological changes, the electrode selection may need adjustment over time. Such adjustment may also be performed manually or automatically. When the exact location of the pain changes, the electronics may be reprogrammed to stimulate other electrode(s) in another cluster and to cause pain relief in the new pain region.

The electrode pad is preferably integrated in a brace or garment to facilitate correct placement of the electrodes. When the use of a brace or garment is combined with the selective stimulation of particular electrodes or electrode clusters, the chance of applying currents to the correct stimulation points is very high. Selection of stimulation points may be done based on electrical skin properties and/or user feedback. Such selection methods are, e.g., described in Philips patent applications WO 1999/052588 Al and WO 2009/138961.

These and other aspects of the invention are apparent from and will be elucidated with reference to the embodiments described hereinafter.

### BRIEF DESCRIPTION OF THE DRAWINGS

In the drawings:
Fig. 1 schematically shows a transcutaneous electro-stimulation device according to the invention,
Fig. 2 shows a square electrode arrangement according to the invention,
Fig. 3 shows a triangular electrode arrangement according to the invention,
Fig. 4 shows a human knee with a stimulation area and a corresponding area for pain relief,
Fig. 5 shows test results of a test for finding stimulation points,
Fig. 6 shows an area for pain relief divided into specific pain regions,
Fig. 7 shows the test results of Fig. 4 plus a relation between stimulation points and pain relief regions,
Fig. 8 shows the test results of Figs. 4 and 7 together with a square grid electrode matrix arrangement,
Fig. 9 shows the test results of Figs. 4 and 7 together with a triangular grid electrode matrix arrangement, and
Fig. 10 shows an exemplary electrode pad according to the invention.

### DETAILED DESCRIPTION OF THE INVENTION

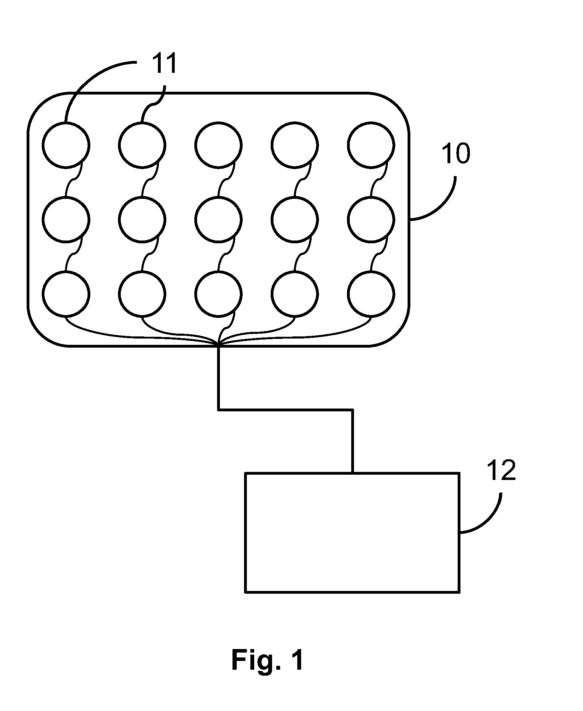

Fig. 1 schematically shows a transcutaneous electro-stimulation device according to the invention. The device comprises an electrode pad 10 with multiple stimulation electrodes 11. The electrodes 11 are arranged in a matrix. In this exemplary embodiment, the matrix is a rectangular grid with 3 by 5 electrodes 11. The electrodes 11 are coupled to an electronic circuit 12. The electronics are configured to apply stimulation currents or voltages to selective electrodes 11 or clusters of electrodes 11.

The electrode pad 10 is applied to human or animal skin at a position where electro-stimulation is planned. Electro-stimulation may be used for different purposes. In the following the use of electro-stimulation for pain relief in a human knee is discussed by way of example. The invention is however not limited to such an application. When a stimulation current or voltage is applied to one or more selected electrodes 11, a stimulation point on the skin area underneath the electrode pad is stimulated. This stimulation causes pain relief in specific parts of the knee. The region where the pain relief occurs is not necessarily directly underneath or close to the stimulation point, but usually is somewhat further away. When one or more electrodes 11 are selected to activate a stimulation point, other electrodes of the electrode pad 10 or an external electrode pad may be used as counter electrodes.

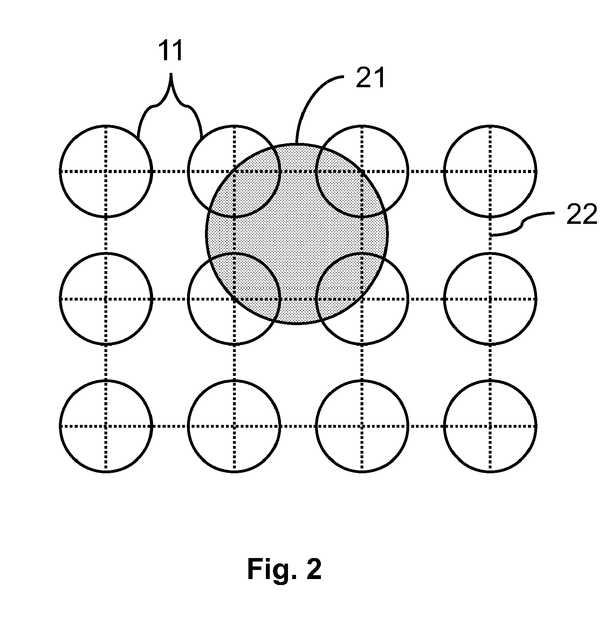

Fig. 2 shows a square electrode arrangement according to the invention. In this example, the electrodes 11 are arranged at the corner points of a square or rectangular grid 22. The arrangement and size of the electrodes 11 is such that a stimulation responsive surface area (=stimulation area) 21 at the skin underneath the electrode pad 10 is at least partly covered by one or more electrodes 11, because the space between two adjacent electrodes 11 is smaller than the diameter of the surface of the stimulation area 21. The stimulation responsive area 21 is not a singular point at the human skin, but a small area at the human skin where stimulation will result in a remote pain relief sensation.

During trial studies it was determined that, in the knee area, the average size of the stimulation areas is around 300 mm², which corresponds to a circular area with a diameter of approximately 20 mm. The 300 mm² surface area is an average value for all tested stimulation points. It is to be noted that some stimulation points may be substantially smaller. It may be decided to ignore such small stimulation points. To ensure that also the smaller stimulation areas 21 are covered by electrodes 11, the distance between the electrodes 11 may be chosen even smaller.

In preferred embodiments, the size and arrangement of the electrodes 11 in the matrix is such that irrespective of the position of the stimulation area 21 relative to the grid 22, the coverage of the stimulation area 21 by electrode surfaces 11 is at least equal to the surface area of one electrode 11. In the example shown in Fig. 2, the minimal overlap is even more than one full electrode surface 11.

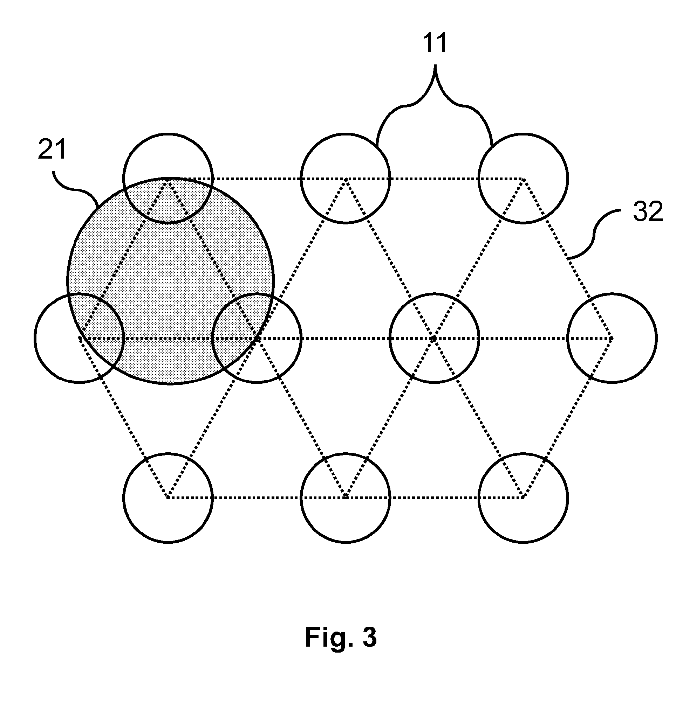

Fig. 3 shows a triangular electrode arrangement according to the invention. In this example, the electrodes 11 are arranged at the corner points of a triangular grid 32. The arrangement and size of the electrodes 11 is such that the stimulation point 21 at the skin underneath the electrode pad 10 is at least partly covered by one or more electrodes 11, because the space between two adjacent electrodes 11 is smaller than the surface area of the stimulation point 21. In this example, the overlap between stimulation area 21 and the electrodes 11 is always at least equal to the surface area of one electrode 11.

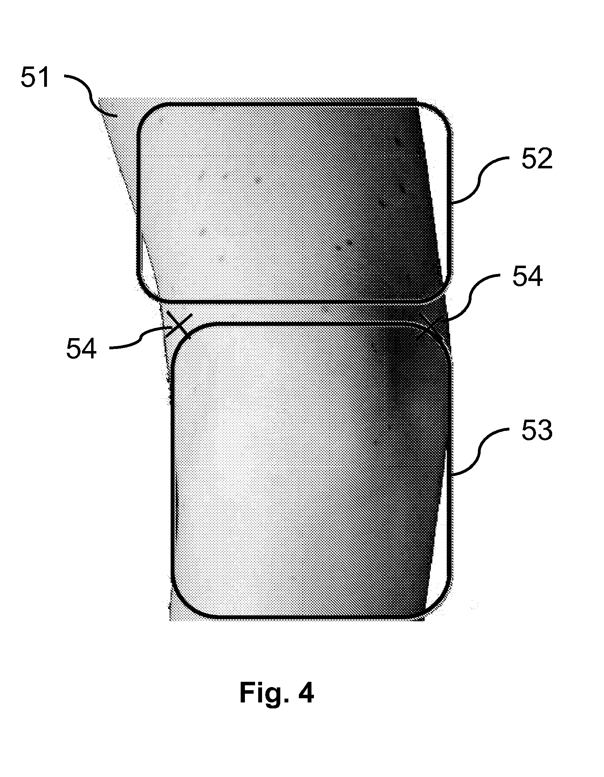

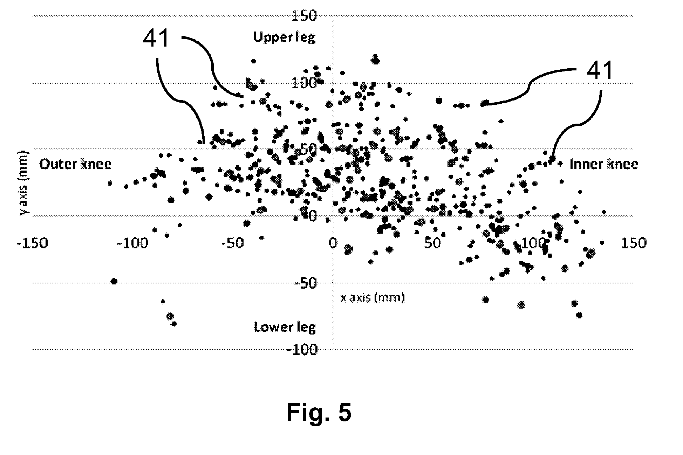

Fig. 4 shows a human knee with a stimulation area 52 and a corresponding area 53 for pain relief. Stimulation points are remote from the pain area and are located slightly different for per individual, but still within a defined area. Fig. 5 shows test results of a test for finding stimulation points 41 in different individuals. The results were obtained in a trial in which 20 subjects were tested. The stimulation points 41 were searched for in the stimulation area 52 which is mainly situated proximal of the knee. During the trial, pain relief was experienced in the pain relief area 53 which is partly situated around the knee and partly distal of the knee. Two extremities 54 of the femur bone are used as reference points for defining the stimulation area 52 and the area 53 for pain relief.

The trial measurements resulted in a dataset of stimulation locations and their accompanying areas in which pain relief occurs. The location and size of the stimulation responsive surface areas were determined. The diagram of Fig. 4 shows the positions of the registered stimulation points 41 for 20 people on three different days. The positions of the stimulation points 41 are defined relative to two axes crossing each other in (0,0) somewhere above the center of the knee. Positive *x* values correspond to positions closer to the inner knee, negative *x* values to positions closer to the outer knee. In a similar way, positive *y* values correspond to positions further away from the lower leg and negative *y* values to positions closer to the lower leg.

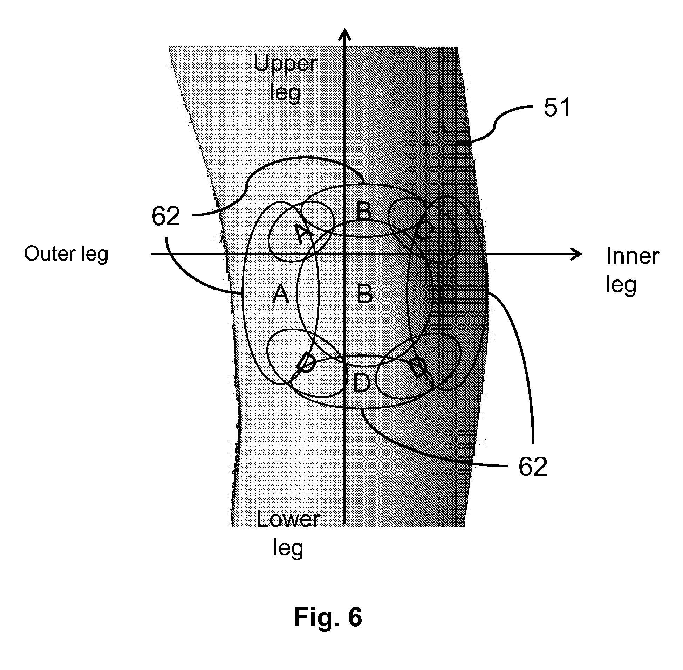

Fig. 6 shows an area for pain relief divided into 4 specific pain regions 62 A, B, C and D. This division into 4 separate regions 62 is just an example. Instead also a different division into 4 regions 62 or a division into a different number of separate regions 62 may be used.

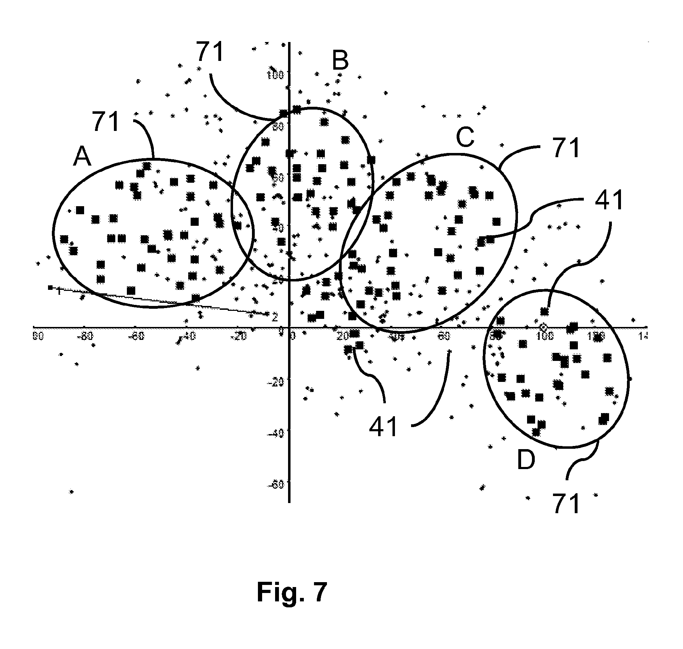

Fig. 7 shows the test results of Fig. 4 plus a relation between stimulation points 41 and pain relief regions 62. In this diagram, the stimulation points 41 of Fig. 4 are clustered with respect to the corresponding pain relief regions 62 A, B, C and D shown in Fig. 6. Four clusters 71 A, B, C and D of stimulation points 41 are found. Most points 41 in each cluster 71 A, B, C and D cause pain relief in the corresponding pain relief region 62 A, B, C and D.

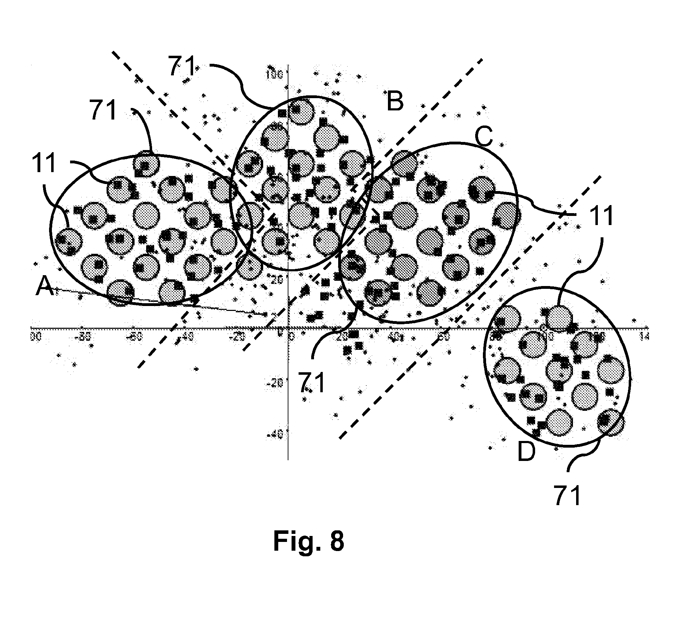

Fig. 8 shows the test results of Figs. 5 and 7 together with a square grid electrode matrix arrangement. Each cluster 71 A, B, C and D is filled with stimulation electrodes 11. These clusters 71 A, B, C and D with stimulation electrodes 11 can be activated separately and selectively for causing pain relief in one or more different pain relief regions 62 A, B, C and D. Activating one cluster 71 means to use at least one of its electrodes 11 as an active stimulation electrode 11. The counter electrode can be one or more electrodes 11 from the same or another cluster 71 or a dedicated counter electrode or cluster of electrodes, not belonging to a stimulation cluster 71.

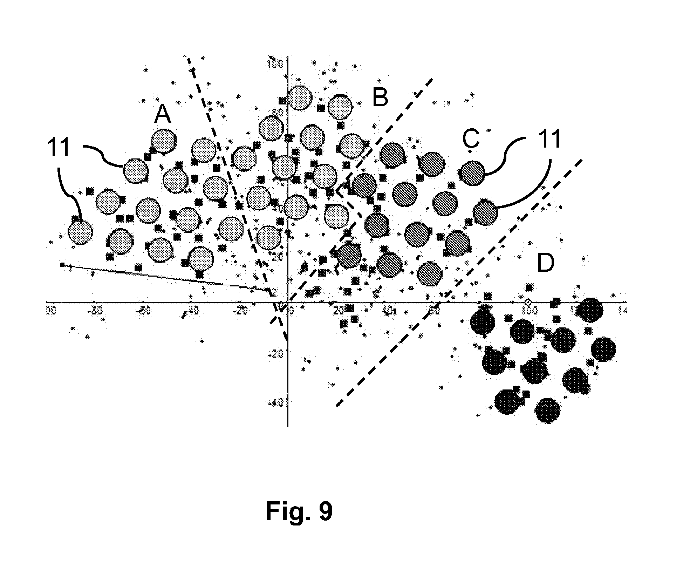

In the alternative embodiments of Fig. 9 a triangular grid electrode matrix arrangement is used. In a preferred embodiment, such a triangular layout is used with an edge to edge distance between the electrodes 11 of approximately 5-10 mm and an electrode diameter of about 10 mm.

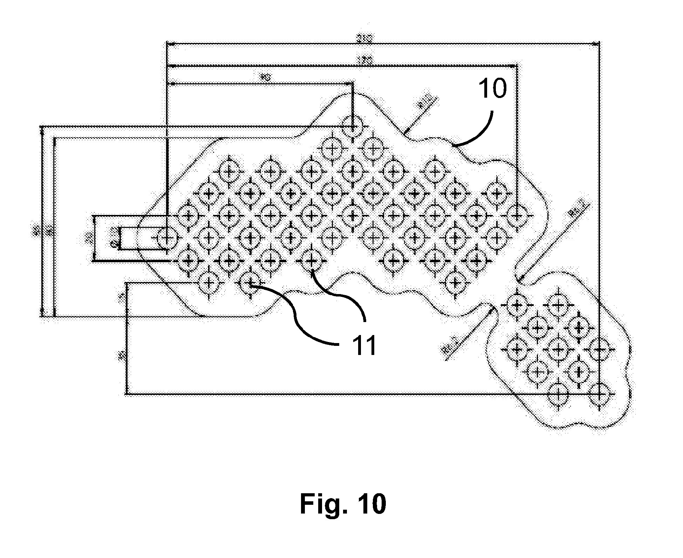

Fig. 10 shows an exemplary electrode pad 10 according to the invention. The electrode pad 10 comprises electrodes 11 in a matrix arrangement. The distances between adjacent electrodes 11 is small enough to ensure coverage of all or most stimulation points on the skin part where the electrode pad 10 is to be attached. The coupling of the electrodes 11 to the electronics 12 is such that stimulation currents or voltages can be applied to selected individual stimulation electrodes 11 or clusters of stimulation electrodes 11. Depending on the pain area(s), the electronic circuit 12 selects one or more stimulation electrodes 11 to activate in the cluster corresponding to the pain area. The electrode 11 selection may involve user interaction. The electrode pad 10 may be integrated into a brace or garment in order to improve the chance of correct placement of the electrode pad 10 on the human or animal skin.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

## Claims

1. A transcutaneous electro-stimulation device comprising:
- a matrix of stimulation electrodes (11) distributed on an electrode pad (10) and being configured to be applied to a skin area of a human or animal body, the skin area covered by the matrix comprising multiple stimulation responsive surface areas (21), and
- an electronic circuit (12) being coupled to the stimulation electrodes (11) and being configured to apply a stimulation signal to at least one of the stimulation electrodes (11) in order to stimulate one of the stimulation responsive surface areas (21),
the arrangement of the stimulation electrodes (11) in the matrix being such that a distance between adjacent stimulation electrodes (11) in the matrix is smaller than a diameter of the stimulation responsive surface areas (21).

2. A transcutaneous electro-stimulation device according to claim 1, wherein the size and the arrangement of the stimulation electrodes (11) in the matrix is such that irrespective of a position of the matrix relative to the stimulation responsive surface area (21), the stimulation responsive surface area (21) is covered by a total amount of electrode surface equal to at least a surface of one of the stimulation electrodes (11).

3. A transcutaneous electro-stimulation device according to claim 1, wherein the stimulation electrodes (11) are arranged in a rectangular grid (22).

4. A transcutaneous electro-stimulation device according to claim 1, wherein the stimulation electrodes (11) are arranged in a triangular grid (32).

5. A transcutaneous electro-stimulation device according to claim 1, wherein the device is arranged for causing pain relief in specific pain regions of the human or animal body.

6. A transcutaneous electro-stimulation device according to claim 5, wherein the stimulation electrodes (11) in the matrix are divided into at least two clusters (71) of stimulation electrodes (11), each cluster (71) being related to one of the specific pain regions.

7. A transcutaneous electro-stimulation device according to claim 1, wherein the device is arranged for causing muscle contraction in specific muscle regions of the human or animal body.

8. A transcutaneous electro-stimulation device according to claim 1, wherein the electronic circuit (12) is configured such that, when the stimulation signal is applied to the at least one of the stimulation electrodes (11), other ones of the stimulation electrodes (11) function as a return electrode.

9. A transcutaneous electro-stimulation device according to claim 1, wherein the electrode pad (10) is integrated in a brace or garment.

10. A transcutaneous electro-stimulation device according to claim 1, wherein a selection of stimulation electrodes (11) to be activated is made based on measured skin properties and/or muscle activity.

## Amended claims

### Amended claims in accordance with Rule 137(2) EPC.

**1.** A transcutaneous electro-stimulation device for causing pain relief in specific pain regions of the human or animal body, the device comprising:
- a matrix of stimulation electrodes (11) distributed on an electrode pad (10) and being configured to be applied to a skin area of a human or animal body, the skin area covered by the matrix comprising multiple stimulation responsive surface areas (21), and
- an electronic circuit (12) being coupled to the stimulation electrodes (11) and being configured to selectively apply a stimulation signal to at least one of the stimulation electrodes (11) in order to stimulate one of the stimulation responsive surface areas (21), wherein
the arrangement of the stimulation electrodes (11) in the matrix are such that a distance between adjacent stimulation electrodes (11) in the matrix is smaller than 20 mm, and wherein the stimulation electrodes (11) in the matrix on the electrode pad (10) are divided into at least two clusters (71) of stimulation electrodes (11), each cluster (71) being related to one of the specific pain regions.

**2.** A transcutaneous electro-stimulation device according to claim 1, wherein the distance between adjacent stimulation electrodes (11) in the matrix is within a range of 5-10 mm.

**3.** A transcutaneous electro-stimulation device according to claim 1, wherein the stimulation electrodes (11) are arranged in a rectangular grid (22).

**4.** A transcutaneous electro-stimulation device according to claim 1, wherein the stimulation electrodes (11) are arranged in a triangular grid (32).

**5.** A transcutaneous electro-stimulation device according to claim 1, wherein the electronic circuit (12) is configured such that, when the stimulation signal is applied to the at least one of the stimulation electrodes (11), other ones of the stimulation electrodes (11) function as a return electrode.

**6.** A transcutaneous electro-stimulation device according to claim 1, wherein the electrode pad (10) is integrated in a brace or garment.

**7.** A transcutaneous electro-stimulation device according to claim 1, further comprising means for determining electrical skin properties and wherein a selection of stimulation electrodes (11) to be activated is made based on the measured electrical skin properties.
